# EUROPEAN PATENT SPECIFICATION

(11) **EP 4 392 577 B1**
(45) Date of publication and mention of the grant of the patent: **26.11.2025**
(21) Application number: 22772802.9
(22) Date of filing: 23.08.2022
(51) Int. Cl.: C12Q 1/6853, C12Q 1/6806, C12N 15/10

(54) **OPTIMISED SET OF OLIGONUCLEOTIDES FOR BULK RNA BARCODING AND SEQUENCING**
OPTIMIERTER SATZ VON OLIGONUKLEOTIDEN ZUR MASSEN-RNA-BARCODIERUNG UND -SEQUENZIERUNG
ENSEMBLE OPTIMISÉ D'OLIGONUCLÉOTIDES POUR LE CODAGE À BARRES ET LE SÉQUENÇAGE D'ARN EN VRAC

(30) Priority: 24.08.2021 WO PCT/EP2021/073346; 07.06.2022 WO PCT/EP2022/065323
(43) Date of publication of application: 03.07.2024
(73) Proprietor: ECOLE POLYTECHNIQUE FEDERALE DE LAUSANNE (EPFL), 1015 Lausanne (CH)
(72) Inventor: DAINESE, Riccardo, 1304 Cossonay-Ville (CH); ALPERN, Daniel, 74500 Evian-les-Bains (FR); GARDEUX, Vincent, 74500 Evian-les-Bains (FR); DEPLANCKE, Bart, 1053 Cugy (CH)
(74) Representative: KATZAROV S.A.
(86) International application number: PCT/EP2022/073458
(87) International publication number: WO 2023/025784

(56) References cited:
- WO-A1-2015/164212
- WO-A1-2016/010856
- WO-A1-2016/040476
- WO-A1-2019/131470
- US-A1- 2020 157 600
- PANU SOMERVUO ET AL: "BARCOSEL: a tool for selecting an optimal barcode set for high-throughput sequencing", BMC BIOINFORMATICS, BIOMED CENTRAL LTD, LONDON, UK, vol. 19, no. 1, 5 July 2018 (2018-07-05), pages 1 - 6, XP021258232, DOI: 10.1186/S12859-018-2262-7
- YANG IN SEOK ET AL: "Development of a program for in silico optimized selection of oligonucleotide-based molecular barcodes", PLOS ONE, vol. 16, no. 2, 18 January 2021 (2021-01-18), pages e0246354, XP055915055, DOI: 10.1371/journal.pone.0246354

## Description

### FIELD OF THE INVENTION

The present invention relates generally to the field of nucleic acid sequencing and provides oligonucleotide molecules and barcodes contained therein. These oligonucleotide molecules and barcodes molecules are useful in sequencing to identify and resolve errors.

### BACKGROUND OF THE INVENTION

RNA sequencing has become the method of choice for genome-wide transcriptomic analyses as its price has substantially decreased over the last years. Nevertheless, the high cost of standard RNA library preparation and the complexity of the underlying data analysis still prevent this approach from becoming as routine as quantitative PCR (qPCR), especially when many samples need to be analyzed.
To alleviate this high cost, the emerging single-cell transcriptomics field implemented the sample barcoding/early multiplexing principle. This reduces both the RNA-seq cost and preparation time by allowing the generation of a single sequencing library that contains multiple distinct samples/cells (Ziegenhain et al., 2017, Mol. Cell 65, 631-643.e4).
Such a strategy could also be of value to reduce the cost and processing time of bulk RNA sequencing of large sets of samples (Kilpinen et al., 2013, Science 342, 744-747; Waszak. et al., 2015, Cell 162, 1039-1050; Pradhan et al. 2017, Sci. Rep. 7, 42130). However, there have been surprisingly few efforts to explicitly adapt and validate the early-stage multiplexing protocols for reliable and affordable profiling of bulk RNA samples.
Early multiplexing protocols designed for single-cell RNA profiling (CEL-seq2, SCRB-seq, and STRT-seq) provide a great capacity for transforming large sets of samples into a unique sequencing library (Hashimshony et al. 2016, Genome Biol., 17, 77; Islam et al., 2012, Nat. Protoc. 7, 813-828; Soumillonet al., 2014, bioRxiv, 003236, doi:10.1101/003236). This is achieved by introducing a sample-specific barcode during the RT reaction using a "molecular tag" carried by either the oligo-dT or the template switch oligo (TSO). After individual samples have been "tagged", they are pooled together, and the remaining steps are performed in bulk, thus shortening the time and cost of library preparation.
Since the tag is introduced to the terminal part of the transcript prior to fragmentation, the reads solely cover the 3' or 5' end of the transcripts. The 3'DGE approach for bulk RNA profiling, has been adopted in several recent studies, such as PLATE-seq (Bush et al., 2017, Nat. Commun. 8, 105), DRUG-seq (Ye et al., 2018, Nat. Commun. 9, 1-9), 3'POOL-seq (Sholder et al., 2020, BMC Genomics 21, 64), PME-seq (Pandey et al., 2020, Nat. Protoc., 15, 1459-1483) and BRB-seq (Alpern et al., 2019 Genome Biol. 20, 71). These techniques have two main commonalities: i) using barcoded DNA oligos used to "tag" poly-adenylated RNA molecules during first strand synthesis and ii) pooling together of all the tagged samples in one tube after the barcoding step.
The overarching goal of these techniques is to decrease the costs and increase the throughput associated with mRNA sequencing library preparation of bulk samples.
This is achieved by reducing reagents, consumables and personnel time through pooling in one solution several barcoded samples. In simple terms, it is much more cost-effective and simpler to process e.g. 100 samples in one tube than 100 samples in 100 tubes.
One of the main challenges of RNA barcoding applied to bulk samples is the ability to guarantee a uniform distribution of sequencing reads across all samples. This challenge is due to the fact that the "molecular barcodes" used during the RNA barcoding step are a functional portion of the "reverse transcription primer" and, as such, different barcodes (i.e. barcodes with different sequences) can have significant effect on the efficiency of the overall workflow. For example, empirical experimental evidence highlights the following potential issues: i) barcodes can lead to unwanted secondary structures that interfere or prevent efficient priming ii) completely random barcodes may end up having very similar or repeating sequences which are then difficult to resolve at the sequencing stage, iii) certain barcodes can be preferentially amplified within the same pool and last, but not least, iv) certain barcodes preferentially bind mitochondrial transcripts, which then appear as an unwanted bias in the sequencing results.
WO 2016/040476 discloses a method called Dropseq using bead bound primers with bead specific barcode made by 12 rounds of reverse-direction phosphoramidite synthesis, a UMI and polyT. This document does not disclose the barcode sequences of the present invention.
US 2020/157600 discloses oligonucleotides for single cell transcriptional profiling with sequencing adapter, cell label barcode, UMI and polyT for capturing of mRNA and cDNA synthesis. This document does not disclose the barcode sequences of the present invention.
WO 2015/164212 discloses droplet encapsulation of single cells together with lysis and RT buffer and a particle comprising releasable barcoded primers that a UMI and polyT region, for expression profiling based on sequencing. The barcode is from a predefined set with error correction based on Hamming distance and the oligonucleotide may furthermore include sequencing adapter tags such as the well known P5 or P7 primer sequences. This document does not disclose the barcode sequences of the present invention.
Therefore, there is still a need for more accurate, dependable sequencing tools, (i) and methods that can eliminate barcoding sample-to-sample variation and (ii) methods that use them to improve various barcoding approaches, including the barcoding-mediated high-accuracy sequencing method.

### SUMMARY OF THE INVENTION

The present invention provides one or more oligonucleotide molecules comprising, from 5' to 3',
a) a sequencing adapter,
b) a barcode sequence consisting of 9 to 15 nucleotides, preferably 12 nucleotides,
   and,
c) an mRNA capture sequence,
wherein the barcode sequence is selected from the group comprising SEQ ID: NO. 1 to 96.

Further provided is a set of oligonucleotide molecules consisting of 96 oligonucleotide molecules, each molecule comprising, from 5' to 3',
a) a sequencing adaptor,
b) a barcode sequence independently selected from the group consisting in SEQ ID: NO. 1 to 96,
   and,
c) an mRNA capture sequence and,
d) optionally a UMI.

Further provided is the use of a set of oligonucleotide molecules of the invention in a sequencing method.

Further provided is a method for providing a cDNA library, the method comprising the steps of
a) Providing a plurality of RNA samples obtained from a biological sample;
b) Contacting separately each RNA sample with one or more oligonucleotide molecules of the invention, or of a library of the invention, or of a barcode oligonucleotide sequence or a combination of one or more thereof, of the invention, under annealing conditions;
c) Incubating separately each sample under reverse transcription reaction conditions;
d) Pooling together all the cDNA:RNA sample;
e) Proceeding to second strand synthesis under synthesis conditions; and
f) Proceeding with tagmentation and amplification under suitable conditions so as to obtain a cDNA library.
Further provided is a method for sequencing RNA, the method comprising the steps of
a) Providing a cDNA library obtained by the method of the invention; and
b) Proceeding to the sequencing under suitable conditions.
Also provided is a kit comprising
i) a set of oligonucleotide molecules of any one of the invention,
ii) a support for sample preparation, such as a 96-well plate, and
iii) reagents for sequencing.
Also provided is the use of the kit of the invention, or of set of oligonucleotide molecules of the invention in a single-cell RNA profiling method.

### DESCRIPTION OF THE FIGURES

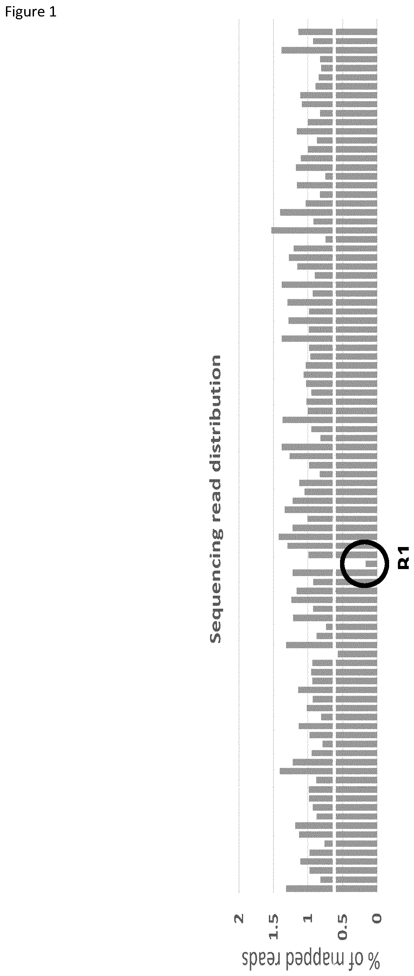

**Figure 1** shows a "bad" example of sequencing read distribution for a suboptimal set of 96 barcodes. As can be seen in the circled area, barcodes can systematically underperform as compared to the others.

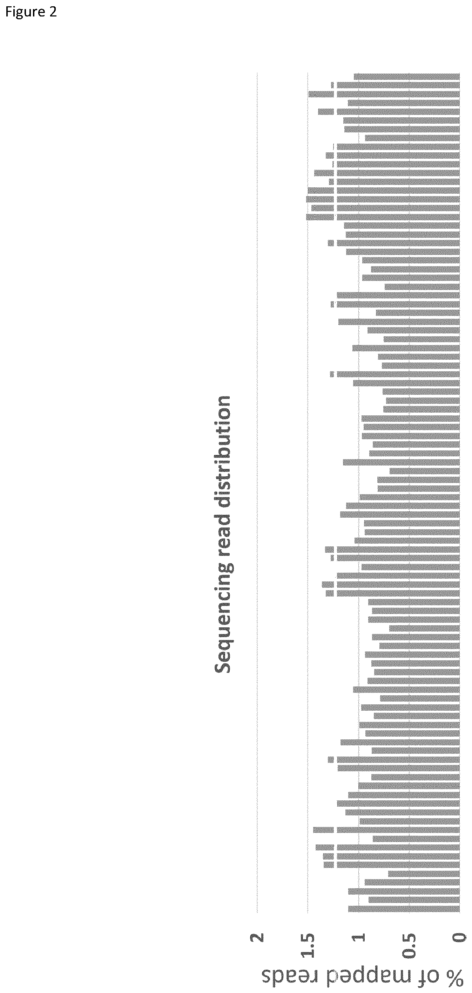

**Figure 2** shows the read distribution of the optimal set of 96 barcodes of the invention in which all barcodes are functional and obtain a similar number of sequencing reads.

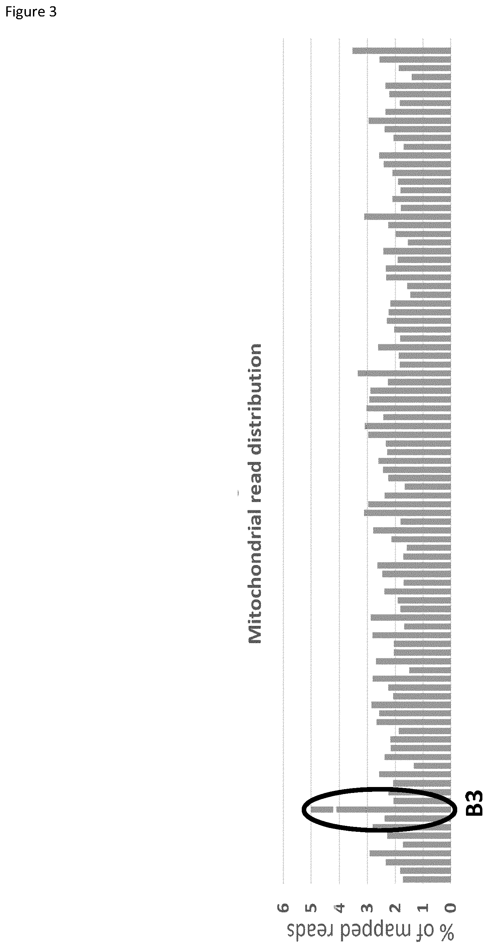

**Figure 3** shows a "bad" example of mitochondrial read distribution for a suboptimal set of 96 barcodes. As can be seen in the circled area, barcodes can systematically obtain more mitochondrial reads as compared to the others.

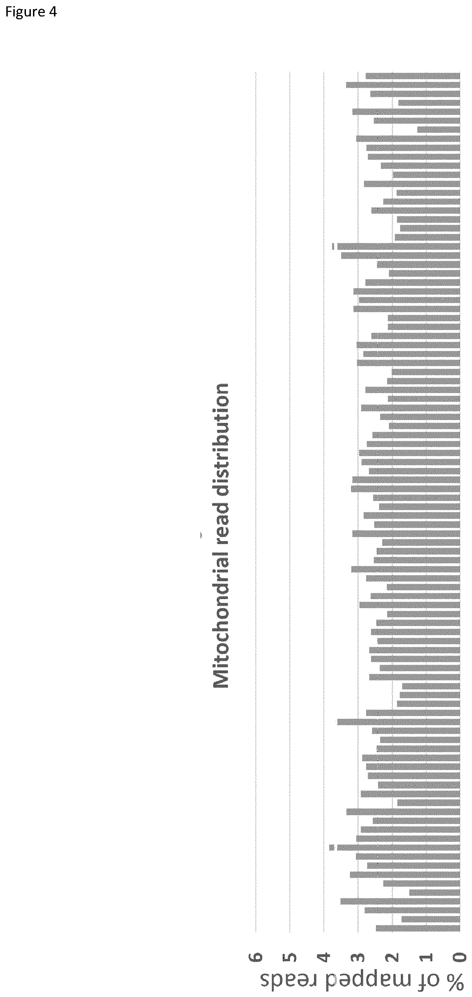

**Figure 4** shows the mitochondrial read distribution of the optimal set of 96 barcodes of the invention in which all barcodes are functional and obtain a similar number of mitochondrial reads.

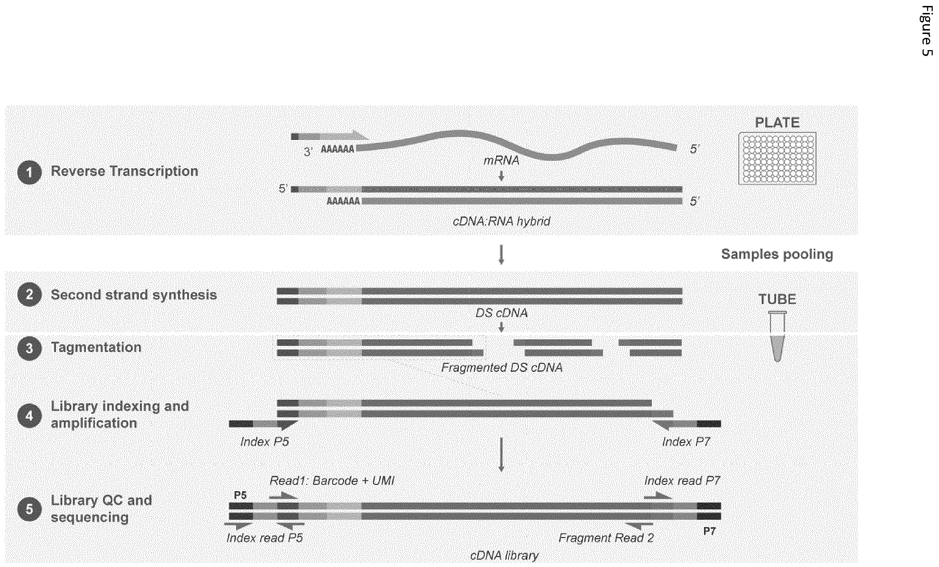

**Figure 5** shows a schematic overview of the method described herein.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of include/including, that is to say permitting the presence of one or more features or components. The terms "comprise(s)" and "comprising" also encompass the more restricted ones "consist(s)", "consisting" as well as "consist/consisting essentially of", respectively.

As used in the specification and claims, the singular form "a", "an" and "the" include plural references unless the context clearly dictates otherwise.

As used herein, "one or more" includes"two or more", "three or more", etc. For example, one or more oligonucleotide molecules refers to one oligonucleotide molecule, two oligonucleotide molecules, three oligonucleotide molecules, *etc....*

The present invention is based on the discovery of an optimal set of 96 barcoded oligonucleotides for multiplexed RNA sequencing. These oligonucleotides contain barcodes that are 12 base pairs long and have been therefore selected from a pool of 4^12 = 16'777'216 potential candidates. This large pool has been filtered twice, first computationally and then experimentally as disclosed herein. The goal was to obtain an optimized set of barcodes for further being able to 1) uniquely demultiplex the samples, with error-tolerance for sequencing errors, and 2) adapt the barcodes for potential technical bias such as overrepresentation of polyT sequences due to preferential amplification of certain sequences.

In one aspect, the invention provides one or more oligonucleotide molecules comprising, from 5' to 3',
a) a sequencing adapter,
b) a barcode sequence consisting of 9 to 15 nucleotides, preferably 12 nucleotides,
   and,
c) an mRNA capture sequence,
wherein the barcode sequence is selected from the group comprising SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20, SEQ ID NO. 21, SEQ ID NO. 22, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38, SEQ ID NO. 39, SEQ ID NO. 40, SEQ ID NO. 41, SEQ ID NO. 42, SEQ ID NO. 43, SEQ ID NO. 44, SEQ ID NO. 45, SEQ ID NO. 46, SEQ ID NO. 47, SEQ ID NO. 48, SEQ ID NO. 49, SEQ ID NO. 50, SEQ ID NO. 51, SEQ ID NO. 52, SEQ ID NO. 53, SEQ ID NO. 54, SEQ ID NO. 55, SEQ ID NO. 56, SEQ ID NO. 57, SEQ ID NO. 58, SEQ ID NO. 59, SEQ ID NO. 60, SEQ ID NO. 61, SEQ ID NO. 62, SEQ ID NO. 63, SEQ ID NO. 64, SEQ ID NO. 65, SEQ ID NO. 66, SEQ ID NO. 67, SEQ ID NO. 68, SEQ ID NO. 69, SEQ ID NO. 70, SEQ ID NO. 71, SEQ ID NO. 72, SEQ ID NO. 73, SEQ ID NO. 74, SEQ ID NO. 75, SEQ ID NO. 76, SEQ ID NO. 77, SEQ ID NO. 78, SEQ ID NO. 79, SEQ ID NO. 80, SEQ ID NO. 81, SEQ ID NO. 82, SEQ ID NO. 83, SEQ ID NO. 84, SEQ ID NO. 85, SEQ ID NO. 86, SEQ ID NO. 87, SEQ ID NO. 88, SEQ ID NO. 89, SEQ ID NO. 90, SEQ ID NO. 91, SEQ ID NO. 92, SEQ ID NO. 93, SEQ ID NO. 94, SEQ ID NO. 95, SEQ ID NO. 96.
In one aspect, the one or more oligonucleotide molecules further comprise a unique molecular identifier (UMI).
"Oligonucleotide" or "polynucleotide," which are used synonymously, means a linear polymer of natural or modified nucleosidic monomers linked by phosphodiester bonds or analogs thereof. The term "oligonucleotide" usually refers to a shorter polymer, e.g., comprising from about 3 to about 100 monomers, and the term "polynucleotide" usually refers to longer polymers, e.g., comprising from about 100 monomers to many thousands of monomers, e.g., 10,000 monomers, or more. Oligonucleotides and polynucleotides may be natural or synthetic. Oligonucleotides and polynucleotides include deoxyribonucleosides, ribonucleosides, and non-natural analogs thereof, such as anomeric forms thereof, peptide nucleic acids (PNAs), and the like, provided that they are capable of specifically binding to a target genome by way of a regular pattern of monomer-to-monomer interactions, such as Watson-Crick type of base pairing, base stacking, Hoogsteen or reverse Hoogsteen types of base pairing, or the like.
The terms "peptide," "protein," and "polypeptide" are used interchangeably to refer to a natural or synthetic molecule comprising two or more amino acids linked by the carboxyl group of one amino acid to the alpha amino group of another.
The term "nucleic acid" refers to a natural or synthetic molecule comprising a single nucleotide or two or more nucleotides linked by a phosphate group at the 3' position of one nucleotide to the 5' end of another nucleotide. The nucleic acid is not limited by length, and thus the nucleic acid can include deoxyribonucleic acid (DNA) or ribonucleic acid (RNA).
"Sequencing" refers to determining the order of nucleotides (base sequences) in a nucleic acid sample, e.g. DNA or RNA. Many techniques are available such as Sanger sequencing and High Throughput Sequencing technologies (HTS). Sanger sequencing may involve sequencing via detection through (capillary) electrophoresis, in which up to 384 capillaries may be sequence analysed in one run. High throughput sequencing involves the parallel sequencing of thousands or millions or more sequences at once. HTS can be defined as Next Generation sequencing, i.e. techniques based on solid phase pyrosequencing or as Next-Next Generation sequencing based on single nucleotide real time sequencing (SMRT). HTS technologies are available such as offered by Roche, Illumina and Applied Biosystems (Life Technologies). Further high throughput sequencing technologies are described by and/or available from Helicos, Pacific Biosciences, Complete Genomics, Ion Torrent Systems, Oxford Nanopore Technologies, Nabsys, ZS Genetics, GnuBio. Each of these sequencing technologies have their own way of preparing samples prior to the actual sequencing step. Depending on the sequencing technology used, amplification steps may be omitted.

As used herein, the term "barcode" refers to a unique oligonucleotide sequence that allows a corresponding nucleic acid base and/or nucleic acid sequence to be identified. In certain aspects, the nucleic acid base and/or nucleic acid sequence is located at a specific position on a larger polynucleotide sequence (e.g., a polynucleotide covalently attached to a bead). In certain aspects, barcodes can each have a length within a range of from 4 to 150 nucleotides. The barcode technology (or barcoding) has been a particularly powerful technique for studying the genetic and functional variations of the target pool and for high-accuracy target DNA sequencing. Each barcode can comprise deoxyribonucleotides, optionally all of the nucleotides in a barcode region are deoxyribonucleotides. One or more of the deoxyribonucleotides may be a modified deoxyribonucleotide (e.g. a deoxyribonucleotide modified with a biotin moiety or a deoxyuracil nucleotide). The barcodes may comprise one or more degenerate nucleotides or sequences. The barcode regions may not comprise any degenerate nucleotides or sequences.

As used herein, the term "sequencing adapter" refers an oligonucleotide sequence that can be used in subsequent sequencing steps (so-called sequencing adapters). Or primers that are used to amplify a subset of fragments prior to sequencing may contain parts within their sequence that introduce sections that can later be used in the sequencing step, for instance by introducing through an amplification step a sequencing adapter or a capturing moiety in an amplicon that can be used in a subsequent sequencing step. Depending also on the sequencing technology used, amplification steps may be omitted.
Any commercially available sequencing adapter can be used, in one aspect, the sequencing adapter comprises, or consists of, CTA CAC GAC GCT CTT CCG ATC (SEQ ID No. 97).

As used herein, a "unique molecular identifier" or UMI is a complex indices added to sequencing libraries before any PCR amplification steps, enabling the accurate bioinformatic identification of PCR duplicates thus enabling to remove PCR duplicates. In one aspect, the UMI is an oligonucleotide sequence consisting of a sequence (N)n(V)m, wherein N is any nucleotide selected from A, T, C and G; V is any nucleotide selected from A, C and G; n is an integer selected from 1 to 20, and m is an integer selected from 1 to 20. Preferably, the UMI comprises, or consists of, SEQ ID NO. 98 (NNNNNNNNNNNVVVVV).

As used herein, an "mRNA capture sequence" is an oligonucleotide sequence that specifically hybridizes to mRNAs. In one aspect, the mRNA capture sequence is a poly-T sequence (10 to 40 T). In a preferred aspect, the mRNA capture sequence is a poly-T sequence (e.g. comprising 10 to 40 T) followed by at least one V and one N, wherein N is any nucleotide selected from A, T, C and G and V is any nucleotide selected from A, C and G. Preferably, the UMI comprises, or consists of, SEQ ID NO. 99 (TTTTTTTTTTTTTTTTTTTTTTTTTTTTTTVN).

"Multiplex sequencing" refers to a sequencing technique that allows for processing a large number of samples on a high-throughput instrument. For multiplex sequencing, individual "barcode" sequences of the invention are added to each sample so that nucleotide sequences from different samples can be distinguished by the unique barcode sequences embedded in each sample. With this technique, multiple DNA or RNA samples can be pooled, processed, sequenced, and analyzed simultaneously.

The present invention further provides a set of oligonucleotide molecules consisting of 96 oligonucleotide, each molecule comprising, from 5' to 3',
a) a sequencing adaptor,
b) a barcode sequence independently selected from the group consisting in SEQ ID: NO. 1 to 96,
   and,
c) an mRNA capture sequence,
as described herein.
In one aspect, the set of oligonucleotide molecules consisting of 96 oligonucleotides further comprises a UMI.

Also provided is the use of one or more oligonucleotide molecules of the invention, or of a set of oligonucleotide molecules of the invention, or of a barcode oligonucleotide sequence, or a combination of one or more thereof, of the invention, in a sequencing method.

The set of oligonucleotide molecules of the invention may be linked by attachment to a solid support (e.g. a bead). A solution of soluble beads (e.g. superparamagnetic beads or styrofoam beads) may be functionalized to enable attachment of two or more oligonucleotide molecules of the invention, set of oligonucleotide molecules of the invention, barcode oligonucleotide sequences, or a combination of one or more thereof,. This functionalization may be enabled through chemical moieties (e.g. carboxylated groups), and/or protein-based adapters (e.g. streptavidin) on the beads. The functionalized beads may be brought into contact with a solution of the above-described molecules under conditions which promote the attachment of two or more molecules to each bead in the solution. Optionally, the molecules are attached through a covalent linkage, or through a (stable) non-covalent linkage such as a streptavidin-biotin bond, or a (stable) oligonucleotide hybridization bond.

The present invention further encompasses a method for providing a cDNA library, the method comprising the step of:
Providing a plurality of RNA samples obtained from a biological sample (step a).
As used herein, the term "biological sample" refers to a tissue (e.g., tissue biopsy), organ, cell (including a cell maintained in culture), cell lysate (or lysate fraction), biomolecule derived from a cell or cellular material (e.g. a polypeptide or nucleic acid), or body fluid from a subject. Non-limiting examples of body fluids include blood, urine, plasma, serum, tears, lymph, bile, cerebrospinal fluid, interstitial fluid, aqueous or vitreous humor, colostrum, sputum, amniotic fluid, saliva, anal and vaginal secretions, perspiration, semen, transudate, exudate, and synovial fluid.
The RNA samples can be obtained from any techniques know in the art. In one aspect, the RNA samples According to a particular aspect, the RNA samples are mRNA samples that can be cell lysates, total DNA/RNA eluate, blood and FFPE tissues.
The method further comprises a step (b) of contacting separately each RNA sample with a set of oligonucleotide molecules of the invention, under annealing conditions.
For examples, RNA samples are thawed on ice, transferred to the corresponding wells of the Oligo-dT primer plat, the plate is then sealed with the AluSeal and placed it in a thermocycler at 65°C for 5 min and immediately put on ice.
The method further comprises a step (c) of incubating separately each sample under reverse transcription reaction conditions.
For example, the RT reaction mix is prepared according to commercial manufacture instruction. Any RT enzyme and buffer commercially available can be used, such as e.g. Lucigen's ERT12910K, ThermoFisher's 18064014 and NEB's M0368S among others.

For example:

| **RT Mix** | **Per well (µL)** |
|---|---|
| RT reaction buffer | 5.0 |
| RT reaction enzyme | 0.4 |
| ddH2O | 4.6 |
| TOTAL | 10.0 |

• Incubate RT reaction mix in thermocycler with the following program:

| **Step** | **Temperature, ° C** | **Time** |
|---|---|---|
| Incubation | 42 | 50 min |
| Inactivation | 70 | 10 min |
| Keep | 4 | pause |

The method further comprises a step (d) of pooling together all the cDNA:RNA sample.
The method further comprises a step (e) of proceeding to second strand synthesis under synthesis conditions such as. This second strand synthesis can be generated by any method known in the art. In one aspect, the second strand synthesis method is selected from the group comprising PCR amplification and nick translation, or a combination thereof.
The method further comprises a step (f) of proceeding with tagmentation, and/or end-repair and ligation and amplification under suitable conditions such as, e.g. the conditions described in the examples, so as to obtain a cDNA library.
The term "biological sample" refers to a tissue (e.g., tissue biopsy), organ, cell (including a cell maintained in culture), cell lysate (or lysate fraction), biomolecule derived from a cell or cellular material (e.g. a polypeptide or nucleic acid), or body fluid from a subject. Non-limiting examples of body fluids include blood, urine, plasma, serum, tears, lymph, bile, cerebrospinal fluid, interstitial fluid, aqueous or vitreous humor, colostrum, sputum, amniotic fluid, saliva, anal and vaginal secretions, perspiration, semen, transudate, exudate, and synovial fluid.
Examples of RNAs include but are not limited to: mRNA, amplicons, rRNA, tRNA, nRNA, siRNA, snRNA, snoRNA, scaRNA, microRNA, dsRNA, ncRNA (e.g. lncRNA), ribozyme, riboswitch and viral RNA (e.g., retroviral RNA).

The present invention further encompasses a method for sequencing RNA, the method comprising the steps of
a) Providing a cDNA library obtained by the method described herein; and
b) Proceeding to the sequencing under suitable conditions such as, e.g. those defined by NGS sequencing providers, which are also known in the art.
The one or more oligonucleotide molecules of the invention may be linked by attachment to a solid support (e.g. a bead).

Also contemplated is one or more kits for performing one or more methods according to the invention. The one or more kit comprising
i) a set of oligonucleotide molecules consisting of 96 oligonucleotide molecules, each molecule comprising, from 5' to 3', a) a sequencing adaptor, b) a barcode sequence independently selected from the group consisting in SEQ ID: 1 to 96, c) optionally a UMI and, d) an mRNA capture sequence,
ii) a support for, such as a 96-well plate, and
iii) reagents for sequencing.
The kit can comprise various molecular biology reagents, including DNA polymerases, RNA polymerases, Reverse-transcriptases, DNA ligases, RNA ligases, transposases, viral integrase, CRISPR/Cas9, zinc finger nucleases, transcription activator-like effector nucleases, exonucleases, endonucleases, Polynucleotide Kinases,nucleotides, oligonucleotides, modified oligonucleotides and optimized buffers.

Further contemplated is the use of the kit of the invention, or of a set of oligonucleotide molecules of the invention, in a single-cell RNA profiling method. Preferably, the single-cell RNA profiling method is similar to the the Bulk RNA Barcoding and sequencing (BRB-seq) method described in Alpern et al., 2019 Genome Biol. 20, 71.

Those skilled in the art will appreciate that the invention described herein is susceptible to variations and modifications other than those specifically described. The foregoing description will be more fully understood with reference to the following Examples.

### EXAMPLES

### Second-strand synthesis

Double-stranded cDNA was generated by either PCR amplification (indicated as PCR in the text) or nick translation (indicated as SSS in the text) [24]. The PCR was performed in 50 µL total reaction volume using 20 µL of pooled and ExoI-treated first-strand reaction, 1 µL of 10 µM LA_oligo (Microsynth) primer, 1 µL of dNTP (0.2mM), 1 µL of with Advantage 2 Polymerase Mix (Clontech, #639206), 5 µL of Advantage 2 PCR buffer, and 22 µL of water following the program (95 °C-1 min; 10 cycles: 95 °C-15 s, 65 °C-30 s, 68 °C-6 min; final elongation at 72 °C-10 min). Alternatively, the second stand was synthesized following the nick translation method. For that, a mix containing 2 µL of RNAse H (NEB, #M0297S), 1 µL of *Escherichia coli* DNA ligase (NEB, #M0205 L), 5 µL of *E. coli* DNA Polymerase (NEB, #M0209 L), 1 µL of dNTP (0 .2mM), 10 µL of 5× Second Stand Buffer (100 mM Tris-HCl (pH 6.9) (AppliChem, #A3452); 25 mM MgCl2 (Sigma, #M2670); 450 mM KCl (AppliChem, #A2939); 0.8 mM β-NAD; 60 mM (NH4)2SO4 (Fisher Scientific Acros, #AC20587); and 11 µL of water was added to 20 µL of ExoI-treated first-strand reaction on ice. The reaction was incubated at 16 °C for 2.5 h or overnight. Full-length double-stranded cDNA was purified with 30 µL (0.6×) of AMPure XP magnetic beads (Beckman Coulter, #A63881) and eluted in 20 µL of water.

### Library preparation and sequencing

The sequencing libraries were prepared by tagmentation of 1-50 ng of full-length double-stranded cDNA. Tagmentation was done either with Illumina Nextera XT kit (Illumina, #FC-131-1024) following the manufacturer's recommendations or with in-house produced Tn5 preloaded with dual (Tn5-A/B) or same adapters (Tn5-B/B) under the following conditions: 1 µL (11 µM) Tn5, 4 µL of 5× TAPS buffer (50 mM TAPS (Sigma, #T5130), and 25 mM MgCl2 (Sigma, #M2670)) in 20 µL total volume. The reaction was incubated 10 min at 55 °C followed by purification with DNA Clean & Concentrator-5 kit (Zymo Research) and elution in 21 µL of water. After that, tagmented library (20 µL) was PCR amplified using 25 µL NEBNext High-Fidelity 2X PCR Master Mix (NEB, #M0541 L), 2.5 µL of P5_BRB primer (5 µM, Microsynth), and 2.5 µL of oligo bearing Illumina index (Idx7N5 5 µM, IDT) using the following program: incubation 72 °C-3 min, denaturation 98 °C-30 s; 10 cycles: 98 °C-10 s, 63 °C-30 s, 72 °C-30 s; final elongation at 72 °C-5 min. The fragments ranging 200-1000 bp were size-selected using AMPure beads (Beckman Coulter, #A63881) (first round 0.5× beads, second 0.7×). The libraries were profiled with High Sensitivity NGS Fragment Analysis Kit (Advanced Analytical, #DNF-474) and measured with Qubit dsDNA HS Assay Kit (Invitrogen, #Q32851) prior to pooling and sequencing using the Illumina NextSeq 500 platform using a custom ReadOne primer (IDT) and the High Output v2 kit (75 cycles) (Illumina, #FC-404-2005). The library loading concentration was 2.2 pM. The read1 sequencing was performed for 6-21 cycles and read2 for 54-70 cycles depending on the experiment.

## Claims

1. A set of oligonucleotide molecules comprising, from 5' to 3',
a) a sequencing adapter,
b) a barcode sequence consisting of 9 to 15 nucleotides, preferably 12 nucleotides, and,
c) an mRNA capture sequence,
wherein the barcode sequence is selected from the group comprising
| | |
|---|---|
| SEQ ID NO. 1 | CTCGAGTAGCAG |
| SEQ ID NO. 2 | CAGCACACGTCA |
| SEQ ID NO. 3 | ACAGCGATCGAC |
| SEQ ID NO. 4 | TAGTGTACGACA |
| SEQ ID NO. 5 | TAGTCGTCTAGC |
| SEQ ID NO. 6 | CATCAGCTGCAC |
| SEQ ID NO. 7 | TAGTAGCACGCA |
| SEQ ID NO. 8 | CAGTCAGCTGAC |
| SEQ ID NO. 9 | CAGCAGTCTACG |
| SEQ ID NO. 10 | CAGCTAGAGCAC |
| SEQ ID NO. 11 | CTAGCATGACGA |
| SEQ ID NO. 12 | ACTCTACGCGAC |
| SEQ ID NO. 13 | CTGTCGAGCTGA |
| SEQ ID NO. 14 | ACAGACGAGTCA |
| SEQ ID NO. 15 | CTATGATCTACG |
| SEQ ID NO. 16 | CTCAGAGCAGAC |
| SEQ ID NO. 17 | ACAGAGACTACG |
| SEQ ID NO. 18 | CTCTGCACTAGC |
| SEQ ID NO. 19 | ACTAGTGACGAC |
| SEQ ID NO. 20 | TACGATGCGTAC |
| SEQ ID NO. 21 | ACGAGACATCAC |
| SEQ ID NO. 22 | CATCACTGCACA |
| SEQ ID NO. 23 | CTGACATCACAG |
| SEQ ID NO. 24 | TAGTACGACTAC |
| SEQ ID NO. 25 | CACGCAGAGTCA |
| SEQ ID NO. 26 | CACACGCATAGC |
| SEQ ID NO. 27 | ACGTATGTCTAG |
| SEQ ID NO. 28 | CATCTCACTAGA |
| SEQ ID NO. 29 | ATCGTCATACGA |
| SEQ ID NO. 30 | TCTAGCACGTGC |
| SEQ ID NO. 31 | TCAGACTGTCAC |
| SEQ ID NO. 32 | TCAGTAGTCTAC |
| SEQ ID NO. 33 | TACTGACACGAC |
| SEQ ID NO. 34 | CATACTCATGAG |
| SEQ ID NO. 35 | CACATGCAGTCG |
| SEQ ID NO. 36 | CACAGATCGAGC |
| SEQ ID NO. 37 | TCGTGACTCAGC |
| SEQ ID NO. 38 | CAGCACGATAGC |
| SEQ ID NO. 39 | CTACAGCACACG |
| SEQ ID NO. 40 | CTGTACGCATGC |
| SEQ ID NO. 41 | CTACACACAGCG |
| SEQ ID NO. 42 | ACTCGTGCGAGA |
| SEQ ID NO. 43 | TAGCGCATGCAC |
| SEQ ID NO. 44 | ACGAGCATCGCA |
| SEQ ID NO. 45 | CTCGCGTACACA |
| SEQ ID NO. 46 | CTGTAGCATCGC |
| SEQ ID NO. 47 | TCTACGTATCGC |
| SEQ ID NO. 48 | CTGAGCTGTACA |
| SEQ ID NO. 49 | CATGCACACAGA |
| SEQ ID NO. 50 | TCATCGACATAG |
| SEQ ID NO. 51 | CACACACTGCGA |
| SEQ ID NO. 52 | CACTGCTAGACA |
| SEQ ID NO. 53 | ACGCGTAGCTAC |
| SEQ ID NO. 54 | CACGTCTATCGC |
| SEQ ID NO. 55 | CAGCATACACGC |
| SEQ ID NO. 56 | CACAGTCGACAC |
| SEQ ID NO. 57 | ACGACGCGTAGA |
| SEQ ID NO. 58 | ATGCGACAGACG |
| SEQ ID NO. 59 | ATCTACTAGTGC |
| SEQ ID NO. 60 | ATCATGAGCAGA |
| SEQ ID NO. 61 | CATCATAGTGAC |
| SEQ ID NO. 62 | CTCGACAGCTCA |
| SEQ ID NO. 63 | ACGCTATCGAGC |
| SEQ ID NO. 64 | CATGCGTCTCAG |
| SEQ ID NO. 65 | ACTGTAGACAGC |
| SEQ ID NO. 66 | TCGCTGCATAGC |
| SEQ ID NO. 67 | CAGTAGCGTGAG |
| SEQ ID NO. 68 | ACTGTCTGTGCA |
| SEQ ID NO. 69 | ACGACAGCATGC |
| SEQ ID NO. 70 | CTCTACATAGAC |
| SEQ ID NO. 71 | CAGCGAGTGACA |
| SEQ ID NO. 72 | CACGACATCAGC |
| SEQ ID NO. 73 | TCATGACGAGAG |
| SEQ ID NO. 74 | ACATCGTAGTCG |
| SEQ ID NO. 75 | CTAGAGATGCGA |
| SEQ ID NO. 76 | TATCAGACATCG |
| SEQ ID NO. 77 | ACACTATGCACA |
| SEQ ID NO. 78 | CAGAGTAGTGCG |
| SEQ ID NO. 79 | CATGACTAGTCG |
| SEQ ID NO. 80 | CTACGTATATGC |
| SEQ ID NO. 81 | CTACGCTCGTAG |
| SEQ ID NO. 82 | CTCGTAGCTAGA |
| SEQ ID NO. 83 | CTACTAGACGCA |
| SEQ ID NO. 84 | TCATGCTCGCGA |
| SEQ ID NO. 85 | ACACGTAGTGCA |
| SEQ ID NO. 86 | ACTGAGCAGCGA |
| SEQ ID NO. 87 | TCACGCGTAGCA |
| SEQ ID NO. 88 | TCATGCACTGCG |
| SEQ ID NO. 89 | ACTGCTATCTCG |
| SEQ ID NO. 90 | CAGACTCTGACG |
| SEQ ID NO. 91 | ACACGCGATACG |
| SEQ ID NO. 92 | TCGACGATGACA |
| SEQ ID NO. 93 | ATACATCGACGA |
| SEQ ID NO. 94 | CACATCACTGAC |
| SEQ ID NO. 95 | TCAGCATACTCA |
| SEQ ID NO. 96 | TATCGTCGCACG. |

2. The set of oligonucleotide molecules of claim 1, further comprising d) a unique molecular identifier (UMI).

3. The set of oligonucleotide molecules of claim 1 or 2, wherein the sequencing adapter comprises, or consists of, CTA CAC GAC GCT CTT CCG ATC (SEQ ID No. 97).

4. The set of oligonucleotide molecules of any one of claims 2 to 3, wherein the UMI consists of a sequence (N)n(V)m, wherein N is any nucleotide selected from A, T, C and G; V is any nucleotide selected from A, C and G; n is an integer selected from 1 to 20, and m is an integer selected from 1 to 20.

5. The set of oligonucleotide molecules of any one of the preceding claims, wherein the mRNA capture sequence is a poly-T sequence followed by at least one V and one N, wherein N is any nucleotide selected from A, T, C and G and V is any nucleotide selected from A, C and G.

6. A set of oligonucleotide molecules consisting of 96 oligonucleotide molecules, each molecule comprising, from 5' to 3',
a) a sequencing adaptor,
b) a barcode sequence independently selected from the group consisting of SEQ ID: NO. 1 to 96,
c) an mRNA capture sequence and,
d) optionally a UMI .

7. Use of a set of oligonucleotide molecules of any one of claims 1 to 6 in a sequencing method.

8. A method for providing a cDNA library, the method comprising the steps of
a) Providing a plurality of RNA samples obtained from a biological sample;
b) Contacting separately each RNA sample with a set of oligonucleotide molecules of any one of claims 1 to 6 under annealing conditions;
c) Incubating separately each sample under reverse transcription reaction conditions;
d) Pooling together all the cDNA:RNA sample;
e) Proceeding to second strand synthesis under synthesis conditions; and
f) Proceeding with tagmentation and/or end-repair and ligation and amplification under suitable conditions so as to obtain a cDNA library.

9. The method for providing a cDNA library of claim 8, wherein the second strand synthesis is generated by a method selected from the group comprising PCR amplification and nick translation, or a combination thereof.

10. A method for sequencing RNA, the method comprising the steps of
a) Providing a cDNA library obtained by the method of claims 8 to 9; and
b) Proceeding to the sequencing under suitable conditions.

11. A kit comprising
i) a set of oligonucleotide molecules of any one of claims 1 to 6
ii) a support for sample preparation, such as a 96-well plate, and
iii) reagents for sequencing.

12. Use of the kit of claim 11, or of set of oligonucleotide molecules of any one of claims 1 to 6 in a single-cell RNA profiling method.

## Patentansprüche

1. Satz von Oligonukleotidmolekülen, der von 5' bis 3' umfasst:
a) einen Sequenzierungsadapter,
b) eine Barcode-Sequenz, die aus 9 bis 15 Nukleotiden, vorzugsweise 12 Nukleotiden, besteht, und,
c) eine mRNA-Capture-Sequenz,
wobei die Barcode-Sequenz ausgewählt ist aus der Gruppe umfassend
| | |
|---|---|
| SEQ ID NO. 1 | CTCGAGTAGCAG |
| SEQ ID NO. 2 | CAGCACACGTCA |
| SEQ ID NO. 3 | ACAGCGATCGAC |
| SEQ ID NO. 4 | TAGTGTACGACA |
| SEQ ID NO. 5 | TAGTCGTCTAGC |
| SEQ ID NO. 6 | CATCAGCTGCAC |
| SEQ ID NO. 7 | TAGTAGCACGCA |
| SEQ ID NO. 8 | CAGTCAGCTGAC |
| SEQ ID NO. 9 | CAGCAGTCTACG |
| SEQ ID NO. 10 | CAGCTAGAGCAC |
| SEQ ID NO. 11 | CTAGCATGACGA |
| SEQ ID NO. 12 | ACTCTACGCGAC |
| SEQ ID NO. 13 | CTGTCGAGCTGA |
| SEQ ID NO. 14 | ACAGACGAGTCA |
| SEQ IDNO. 15 | CTATGATCTACG |
| SEQ ID NO. 16 | CTCAGAGCAGAC |
| SEQ ID NO. 17 | ACAGAGACTACG |
| SEQ ID NO. 18 | CTCTGCACTAGC |
| SEQ ID NO. 19 | ACTAGTGACGAC |
| SEQ ID NO. 20 | TACGATGCGTAC |
| SEQ ID NO. 21 | ACGAGACATCAC |
| SEQ ID NO. 22 | CATCACTGCACA |
| SEQ ID NO. 23 | CTGACATCACAG |
| SEQ ID NO. 24 | TAGTACGACTAC |
| SEQ ID NO. 25 | CACGCAGAGTCA |
| SEQ ID NO. 26 | CACACGCATAGC |
| SEQ ID NO. 27 | ACGTATGTCTAG |
| SEQ ID NO. 28 | CATCTCACTAGA |
| SEQ ID NO. 29 | ATCGTCATACGA |
| SEQ ID NO. 30 | TCTAGCACGTGC |
| SEQ IDNO. 31 | TCAGACTGTCAC |
| SEQ ID NO. 32 | TCAGTAGTCTAC |
| SEQ ID NO. 33 | TACTGACACGAC |
| SEQ ID NO. 34 | CATACTCATGAG |
| SEQ ID NO. 35 | CACATGCAGTCG |
| SEQ ID NO. 36 | CACAGATCGAGC |
| SEQ ID NO. 37 | TCGTGACTCAGC |
| SEQ ID NO. 38 | CAGCACGATAGC |
| SEQ ID NO. 39 | CTACAGCACACG |
| SEQ ID NO. 40 | CTGTACGCATGC |
| SEQ ID NO. 41 | CTACACACAGCG |
| SEQ ID NO. 42 | ACTCGTGCGAGA |
| SEQ ID NO. 43 | TAGCGCATGCAC |
| SEQ ID NO. 44 | ACGAGCATCGCA |
| SEQ ID NO. 45 | CTCGCGTACACA |
| SEQ ID NO. 46 | CTGTAGCATCGC |
| SEQ ID NO. 47 | TCTACGTATCGC |
| SEQ ID NO. 48 | CTGAGCTGTACA |
| SEQ ID NO. 49 | CATGCACACAGA |
| SEQ ID NO. 50 | TCATCGACATAG |
| SEQ IDNO. 51 | CACACACTGCGA |
| SEQ ID NO. 52 | CACTGCTAGACA |
| SEQ ID NO. 53 | ACGCGTAGCTAC |
| SEQ ID NO. 54 | CACGTCTATCGC |
| SEQ ID NO. 55 | CAGCATACACGC |
| SEQ ID NO. 56 | CACAGTCGACAC |
| SEQ ID NO. 57 | ACGACGCGTAGA |
| SEQ ID NO. 58 | ATGCGACAGACG |
| SEQ ID NO. 59 | ATCTACTAGTGC |
| SEQ ID NO. 60 | ATCATGAGCAGA |
| SEQ ID NO. 61 | CATCATAGTGAC |
| SEQ ID NO. 62 | CTCGACAGCTCA |
| SEQ ID NO. 63 | ACGCTATCGAGC |
| SEQ ID NO. 64 | CATGCGTCTCAG |
| SEQ ID NO. 65 | ACTGTAGACAGC |
| SEQ ID NO. 66 | TCGCTGCATAGC |
| SEQ ID NO. 67 | CAGTAGCGTGAG |
| SEQ ID NO. 68 | ACTGTCTGTGCA |
| SEQ ID NO. 69 | ACGACAGCATGC |
| SEQ ID NO. 70 | CTCTACATAGAC |
| SEQ ID NO. 71 | CAGCGAGTGACA |
| SEQ ID NO. 72 | CACGACATCAGC |
| SEQ ID NO. 73 | TCATGACGAGAG |
| SEQ ID NO. 74 | ACATCGTAGTCG |
| SEQ ID NO. 75 | CTAGAGATGCGA |
| SEQ ID NO. 76 | TATCAGACATCG |
| SEQ ID NO. 77 | ACACTATGCACA |
| SEQ ID NO. 78 | CAGAGTAGTGCG |
| SEQ ID NO. 79 | CATGACTAGTCG |
| SEQ ID NO. 80 | CTACGTATATGC |
| SEQ ID NO. 81 | CTACGCTCGTAG |
| SEQ ID NO. 82 | CTCGTAGCTAGA |
| SEQ ID NO. 83 | CTACTAGACGCA |
| SEQ ID NO. 84 | TCATGCTCGCGA |
| SEQ ID NO. 85 | ACACGTAGTGCA |
| SEQ ID NO. 86 | ACTGAGCAGCGA |
| SEQ ID NO. 87 | TCACGCGTAGCA |
| SEQ ID NO. 88 | TCATGCACTGCG |
| SEQ ID NO. 89 | ACTGCTATCTCG |
| SEQ ID NO. 90 | CAGACTCTGACG |
| SEQ ID NO. 91 | ACACGCGATACG |
| SEQ ID NO. 92 | TCGACGATGACA |
| SEQ ID NO. 93 | ATACATCGACGA |
| SEQ ID NO. 94 | CACATCACTGAC |
| SEQ ID NO. 95 | TCAGCATACTCA |
| SEQ ID NO. 96 | TATCGTCGCACG. |

2. Satz von Oligonukleotidmolekülen nach Anspruch 1, ferner umfassend d) einen eindeutigen molekularen Identifikator (UMI).

3. Satz von Oligonukleotidmolekülen nach Anspruch 1 oder 2, wobei der Sequenzierungsadapter CTA CAC GAC GCT CTT CCG ATC (SEQ ID No. 97) umfasst oder daraus besteht.

4. Satz von Oligonukleotidmolekülen nach einem der Ansprüche 2 bis 3, wobei der UMI aus einer Sequenz (N)n(V)m besteht, wobei N ein beliebiges Nukleotid ausgewählt aus A, T, C und G ist; V ein beliebiges Nukleotid ausgewählt aus A, C und G ist; n eine ganze Zahl ausgewählt aus 1 bis 20 ist und m eine ganze Zahl ausgewählt aus 1 bis 20 ist.

5. Satz von Oligonukleotidmolekülen nach einem der vorhergehenden Ansprüche, wobei die mRNA-Capture-Sequenz eine Poly-T-Sequenz ist, gefolgt von mindestens einem V und einem N, wobei N ein beliebiges Nukleotid ausgewählt aus A, T, C und G ist und V ein beliebiges Nukleotid ausgewählt aus A, C und G ist.

6. Satz von Oligonukleotidmolekülen, bestehend aus 96 Oligonukleotidmolekülen, wobei jedes Molekül von 5' bis 3' umfasst:
a) einen Sequenzierungsadapter,
b) eine Barcode-Sequenz unabhängig ausgewählt aus der Gruppe bestehend aus SEQ ID NO. 1 bis 96,
c) eine mRNA-Capture-Sequenz und
d) gegebenenfalls einen UMI.

7. Verwendung eines Satzes von Oligonukleotidmolekülen nach einem der Ansprüche 1 bis 6 in einem Sequenzierungsverfahren.

8. Verfahren zur Bereitstellung einer cDNA-Bibliothek, wobei das Verfahren die folgenden Schritte umfasst:
a) Bereitstellen einer Vielzahl von RNA-Proben, die aus einer biologischen Probe erhalten wurden;
b) Separates Inkontaktbringen jeder RNA-Probe mit einem Satz von Oligonukleotidmolekülen nach einem der Ansprüche 1 bis 6 unter Annealing-Bedingungen;
c) Separates Inkubieren jeder Probe unter Reaktionsbedingungen für die reverse Transkription;
d) Zusammenführen aller cDNA:RNA-Proben in einem Pool;
e) Fortsetzen mit der Synthese des zweiten Strangs unter Synthesebedingungen; und
f) Fortsetzung der Tagmentierung und/oder Endreparatur sowie Ligation und Amplifikation unter geeigneten Bedingungen, um eine cDNA-Bibliothek zu erhalten.

9. Verfahren zur Bereitstellung einer cDNA-Bibliothek nach Anspruch 8, wobei die Synthese des zweiten Strangs durch ein Verfahren erzeugt wird, das aus der Gruppe ausgewählt ist, die PCR-Amplifikation und Nick-Translation oder eine Kombination davon umfasst.

10. Verfahren zur Sequenzierung von RNA, wobei das Verfahren die folgenden Schritte umfasst:
a) Bereitstellen einer cDNA-Bibliothek, die durch das Verfahren nach den Ansprüchen 8 bis 9 erhalten wurde; und
b) Fortsetzen mit der Sequenzierung unter geeigneten Bedingungen.

11. Kit umfassend
i) einen Satz von Oligonukleotidmolekülen nach einem der Ansprüche 1 bis 6,
ii) einen Träger für die Probenvorbereitung, wie etwa eine 96-Well-Platte, und
iii) Reagenzien für die Sequenzierung.

12. Verwendung des Kits nach Anspruch 11 oder eines Satzes von Oligonukleotidmolekülen nach einem der Ansprüche 1 bis 6 in einem Verfahren zur Einzelzell-RNA-Profilierung.

## Revendications

1. Ensemble de molécules oligonucléotidiques comprenant, de 5' à 3',
a) un adaptateur de séquençage,
b) une séquence de code à barres constituée de 9 à 15 nucléotides, de préférence 12 nucléotides, et,
c) une séquence de capture d'ARNm,
dans lequel la séquence de code à barres est choisie dans le groupe comprenant
| | |
|---|---|
| SEQ ID NO. 1 | CTCGAGTAGCAG |
| SEQ ID NO. 2 | CAGCACACGTCA |
| SEQ ID NO. 3 | ACAGCGATCGAC |
| SEQ ID NO. 4 | TAGTGTACGACA |
| SEQ ID NO. 5 | TAGTCGTCTAGC |
| SEQ ID NO. 6 | CATCAGCTGCAC |
| SEQ ID NO. 7 | TAGTAGCACGCA |
| SEQ ID NO. 8 | CAGTCAGCTGAC |
| SEQ ID NO. 9 | CAGCAGTCTACG |
| SEQ ID NO. 10 | CAGCTAGAGCAC |
| SEQ ID NO. 11 | CTAGCATGACGA |
| SEQ ID NO. 12 | ACTCTACGCGAC |
| SEQ ID NO. 13 | CTGTCGAGCTGA |
| SEQ ID NO. 14 | ACAGACGAGTCA |
| SEQ ID NO. 15 | CTATGATCTACG |
| SEQ ID NO. 16 | CTCAGAGCAGAC |
| SEQ ID NO. 17 | ACAGAGACTACG |
| SEQ ID NO. 18 | CTCTGCACTAGC |
| SEQ ID NO. 19 | ACTAGTGACGAC |
| SEQ ID NO. 20 | TACGATGCGTAC |
| SEQ ID NO. 21 | ACGAGACATCAC |
| SEQ ID NO. 22 | CATCACTGCACA |
| SEQ ID NO. 23 | CTGACATCACAG |
| SEQ ID NO. 24 | TAGTACGACTAC |
| SEQ ID NO. 25 | CACGCAGAGTCA |
| SEQ ID NO. 26 | CACACGCATAGC |
| SEQ ID NO. 27 | ACGTATGTCTAG |
| SEQ ID NO. 28 | CATCTCACTAGA |
| SEQ ID NO. 29 | ATCGTCATACGA |
| SEQ ID NO. 30 | TCTAGCACGTGC |
| SEQ ID NO. 31 | TCAGACTGTCAC |
| SEQ ID NO. 32 | TCAGTAGTCTAC |
| SEQ ID NO. 33 | TACTGACACGAC |
| SEQ ID NO. 34 | CATACTCATGAG |
| SEQ ID NO. 35 | CACATGCAGTCG |
| SEQ ID NO. 36 | CACAGATCGAGC |
| SEQ ID NO. 37 | TCGTGACTCAGC |
| SEQ ID NO. 38 | CAGCACGATAGC |
| SEQ ID NO. 39 | CTACAGCACACG |
| SEQ ID NO. 40 | CTGTACGCATGC |
| SEQ ID NO. 41 | CTACACACAGCG |
| SEQ ID NO. 42 | ACTCGTGCGAGA |
| SEQ ID NO. 43 | TAGCGCATGCAC |
| SEQ ID NO. 44 | ACGAGCATCGCA |
| SEQ ID NO. 45 | CTCGCGTACACA |
| SEQ ID NO. 46 | CTGTAGCATCGC |
| SEQ ID NO. 47 | TCTACGTATCGC |
| SEQ ID NO. 48 | CTGAGCTGTACA |
| SEQ ID NO. 49 | CATGCACACAGA |
| SEQ ID NO. 50 | TCATCGACATAG |
| SEQ ID NO. 51 | CACACACTGCGA |
| SEQ ID NO. 52 | CACTGCTAGACA |
| SEQ ID NO. 53 | ACGCGTAGCTAC |
| SEQ ID NO. 54 | CACGTCTATCGC |
| SEQ ID NO. 55 | CAGCATACACGC |
| SEQ ID NO. 56 | CACAGTCGACAC |
| SEQ ID NO. 57 | ACGACGCGTAGA |
| SEQ ID NO. 58 | ATGCGACAGACG |
| SEQ ID NO. 59 | ATCTACTAGTGC |
| SEQ ID NO. 60 | ATCATGAGCAGA |
| SEQ ID NO. 61 | CATCATAGTGAC |
| SEQ ID NO. 62 | CTCGACAGCTCA |
| SEQ ID NO. 63 | ACGCTATCGAGC |
| SEQ ID NO. 64 | CATGCGTCTCAG |
| SEQ ID NO. 65 | ACTGTAGACAGC |
| SEQ ID NO. 66 | TCGCTGCATAGC |
| SEQ ID NO. 67 | CAGTAGCGTGAG |
| SEQ ID NO. 68 | ACTGTCTGTGCA |
| SEQ ID NO. 69 | ACGACAGCATGC |
| SEQ ID NO. 70 | CTCTACATAGAC |
| SEQ ID NO. 71 | CAGCGAGTGACA |
| SEQ ID NO. 72 | CACGACATCAGC |
| SEQ ID NO. 73 | TCATGACGAGAG |
| SEQ ID NO. 74 | ACATCGTAGTCG |
| SEQ ID NO. 75 | CTAGAGATGCGA |
| SEQ ID NO. 76 | TATCAGACATCG |
| SEQ ID NO. 77 | ACACTATGCACA |
| SEQ ID NO. 78 | CAGAGTAGTGCG |
| SEQ ID NO. 79 | CATGACTAGTCG |
| SEQ ID NO. 80 | CTACGTATATGC |
| SEQ ID NO. 81 | CTACGCTCGTAG |
| SEQ ID NO. 82 | CTCGTAGCTAGA |
| SEQ ID NO. 83 | CTACTAGACGCA |
| SEQ ID NO. 84 | TCATGCTCGCGA |
| SEQ ID NO. 85 | ACACGTAGTGCA |
| SEQ ID NO. 86 | ACTGAGCAGCGA |
| SEQ ID NO. 87 | TCACGCGTAGCA |
| SEQ ID NO. 88 | TCATGCACTGCG |
| SEQ ID NO. 89 | ACTGCTATCTCG |
| SEQ ID NO. 90 | CAGACTCTGACG |
| SEQ ID NO. 91 | ACACGCGATACG |
| SEQ ID NO. 92 | TCGACGATGACA |
| SEQ ID NO. 93 | ATACATCGACGA |
| SEQ ID NO. 94 | CACATCACTGAC |
| SEQ ID NO. 95 | TCAGCATACTCA |
| SEQ ID NO. 96 | TATCGTCGCACG. |

2. Ensemble de molécules oligonucléotidiques selon la revendication 1, comprenant en outre d) un identifiant moléculaire unique (UMI).

3. Ensemble de molécules oligonucléotidiques selon la revendication 1 ou 2, dans lequel l'adaptateur de séquençage comprend ou est constitué de CTA CAC GAC GCT CTT CCG ATC (SEQ ID No. 97).

4. Ensemble de molécules oligonucléotidiques selon l'une quelconque des revendications 2 et 3, dans lequel l'UMI est constitué d'une séquence (N)n(V)m, où N est un nucléotide quelconque choisi parmi A, T, C et G ; V est un nucléotide quelconque choisi parmi A, C et G ; n est un nombre entier choisi entre 1 et 20, et m est un nombre entier choisi entre 1 et 20.

5. Ensemble de molécules oligonucléotidiques selon l'une quelconque des revendications précédentes, dans lequel la séquence de capture d'ARNm est une séquence poly-T suivie d'au moins un V et un N, où N est un nucléotide quelconque choisi parmi A, T, C et G et V est un nucléotide quelconque choisi parmi A, C et G.

6. Ensemble de molécules oligonucléotidiques constitué de 96 molécules oligonucléotidiques, chaque molécule comprenant, de 5' à 3',
a) un adaptateur de séquençage,
b) une séquence de code à barres choisie indépendamment dans le groupe constitué par SEQ ID : NO. 1 à 96,
c) une séquence de capture d'ARNm et,
d) éventuellement un UMI.

7. Utilisation d'un ensemble de molécules oligonucléotidiques selon l'une quelconque des revendications 1 à 6 dans un procédé de séquençage.

8. Procédé pour fournir une banque d'ADNc, le procédé comprenant les étapes consistant à
a) fournir une pluralité d'échantillons d'ARN obtenus à partir d'un échantillon biologique ;
b) mettre en contact séparément chaque échantillon d'ARN avec un ensemble de molécules oligonucléotidiques selon l'une quelconque des revendications 1 à 6 dans des conditions d'hybridation ;
c) incuber séparément chaque échantillon dans des conditions de réaction de transcription inverse ;
d) regrouper tout l'échantillon d'ADNc : ARN ;
e) procéder à la synthèse du second brin dans des conditions de synthèse ; et
f) procéder à la tagmentation et/ou à la réparation d'extrémité et à la ligature et à l'amplification dans des conditions appropriées afin d'obtenir une banque d'ADNc.

9. Procédé pour fournir une banque d'ADNc selon la revendication 8, dans lequel la synthèse du second brin est générée par un procédé choisi dans le groupe comprenant l'amplification par PCR et la translation de coupure, ou une combinaison de celles-ci.

10. Procédé de séquençage d'ARN, le procédé comprenant les étapes consistant à
a) fournir une banque d'ADNc obtenue par le procédé selon les revendications 8 et 9 ; et
b) procéder au séquençage dans des conditions appropriées.

11. Kit comprenant
i) un ensemble de molécules oligonucléotidiques selon l'une quelconque des revendications 1 à 6
ii) un support pour la préparation d'échantillon, tel qu'une plaque à 96 puits, et
iii) des réactifs pour le séquençage.

12. Utilisation du kit selon la revendication 11, ou d'un ensemble de molécules oligonucléotidiques selon l'une quelconque des revendications 1 à 6, dans un procédé de profilage d'ARN unicellulaire.
